Europäisches Patentamt

European Patent Office

Office européen des brevets

(11) Publication number: **0 134 452**
**A2**

(12) **EUROPEAN PATENT APPLICATION**

(21) Application number: **84107414.9**

(22) Date of filing: **27.06.84**

(51) Int. Cl.⁴: **A 61 K 7/09**

(30) Priority: **30.06.83 US 509649**

(43) Date of publication of application:
**20.03.85 Bulletin 85/12**

(84) Designated Contracting States:
**AT BE CH DE FR GB IT LI LU NL SE**

(71) Applicant: **Helene Curtis Industries, Inc.**
**325 North Wells Street**
**Chicago Illinois 60601(US)**

(72) Inventor: **Hsiung, Du Y.**
**130 Illinois Street**
**Park Forest Illinois 60466(US)**

(72) Inventor: **Rathnam, Jayaseelan**
**950 Beau Drive**
**Des Plaines Illinois 60016(US)**

(72) Inventor: **Branch, Billye J.**
**17507 Cypress Aveneu**
**Country Club Hills Illinois 60477(US)**

(74) Representative: **Groening, Hans Wilhelm, Dipl.-Ing.**
**Siebertstrasse 4 Postfach 860 340**
**D-8000 München 86(DE)**

(54) Permanent wave neutralizer composition and process.

(57) This invention relates to a new permanent wave neutralizer and to methods of permanently waving hair. More particularly, the neutralizer comprises an oxidizing agent in combination with a polyvalent metal salt selected from water-dispersible, non-toxic salts of aluminum, zirconium, aluminum zirconium coordination complexes and mixtures thereof.

EP 0 134 452 A2

Croydon Printing Company Ltd.

## PERMANENT WAVE NEUTRALIZER
## COMPOSITION AND PROCESS

Technical Field

This invention relates to treating human hair for purposes of permanently waving the hair, and more particularly to aqueous hair treating compositions containing aluminum and zirconium salts and an oxidizing agent for use as permanent wave neutralizers.

Background Art

The practice of chemically treating human hair with keratin disulfide-bond breaking agents, such as thiol and sulfite salts, for "permanently" waving hair is well known in the art. As usually practiced, the conventional process of permanently waving hair usually requires two steps. In the first step, a keratin modifying composition containing a reductive keratin disulfide-bond breaking agent is applied to the hair to form partially reduced hair. This composition is allowed to remain in contact with the hair until the desired amount of softening is effected while holding the softened hair in the new desired wave configuration. In order to permanently set or fix the hair in the new configuration, the keratin modifying composition is usually rinsed out, and, in a second step, a composition containing a keratin disulfide-bond rebuilding agent is applied to form new disulfide bonds in the hair, replacing those broken in the first step. This method of fixing the permanent wave is called neutralization.

The effective concentration of conventional reductive and neutralizing agents within the aforementioned compositions is well known to those skilled in the hair waving arts and need not be discussed herein.

Although neutralization restores strength to the chemically softened hair, the permanence of the new wave configuration is known to be weakened by repeated washings, combing and setting, and by other chemically reactive hair treatments, such as tints. Also, the tightness of the hair style set is weakened under atmospheric high humidity conditions and curled hair tends to relax its set and straighten. Conversely, naturally wavy hair which has been chemically straightened tends to re-kink and get frizzy under high humidity conditions.

This invention provides an improved method for permanently waving hair and improved hair treating compositions for use as permanent wave neutralizers. Hair that has been neutralized with compositions of this invention have long-lasting wave configurations that maintain their set under conditions that normally weaken and relax curl tightness. More particularly, the compositions of this invention restore hydrophobicity to hair, a characteristic of normal hair that is destroyed when the hair is chemically treated with conventional waving and tinting products. Additionally, hair that is neutralized with the compositions of this invention is stronger, less porous, more bodied and has better wave configurations than is hair waved in the conventional manner and neutralized with conventional neutralizers.

The compositions of this invention employ conventional oxidizing agents known in the waving art such as bromates, and hydrogen peroxide, in combination with a water-dispersible, non-toxic polyvalent metal salt.

Mixtures of oxidizing agent and watersoluble polyvalent salts of magnesium, aluminum and calcium are taught by Reiss et al. in U.S. Patent No. 3,266,994 as neutralizer solutions that reportedly produced long lasting waves with a lesser tendency for the fibers to separate from one another. In order to achieve this effect, however, a concentration of at least 18 weight percent metal salt was taught. Magnesium and calcium salts, two of the three materials taught by Reiss, are ineffective when practiced as taught by this invention, as explained below, even at the concentrations practiced by Reiss et al. The improved waving effects achieved with aluminum salts, as practiced herein, surprisingly are achieved at concentrations as low as 0.01 weight percent, several orders of magnitude below those taught by Reiss et al.

Prior attempts to improve the body of waved hair, as taught by Patel in U.S. Patent No. 3,981,312, involved contacting the hair with a calcium, magnesium or aluminum salt in combination with a water soluble reactive polymer resin in a prebonding intervening step of the permanent wave process, prior to neutralization. The Patel teaching also required a high concentration of salt from about 20 weight percent to saturation and contained no oxidizing agent. As mentioned above, calcium and magnesium salts are inoperative when practiced as disclosed herein (in the absence of Patel's resin) in a 2-step procedure. Also, the practice of this invention using aluminum salts requires no prebonding step and is accomplished in a conventional 2-step procedure using low salt concentrations.

-4-

Umezawa and Hazaka in U.S. Patent No. 3,840,401 teach the use of water soluble compounds of calcium, tin, aluminum, lanthanum and zirconium for forming stable metal complexes in hair keratin protein which has been alkali-treated at a pH value in the range of 9.0 to 10.5. However, alkali setting treatments occur primarily by transforming the disulfide bonds in hair keratin cystine groups into lanthionine groups. Such alkali treatments do not require an oxidizing neutralizer, and are unlike conventional waving treatments practiced by this invention. Thus, Umezawa's teachings are practiced in the absence of oxidizing agents. Indeed, oxidizing agents would only weaken the alkali treated hair further and probably destroy it.

It is well known that when hair is frequently tinted, bleached and/or waved, it is weakened and becomes porous. As the porosity of the hair increases, the hair tends to swell and stretch more easily, especially when wet. It is, thus, more vulnerable to breaking during subsequent grooming treatments. An ideal waving product, therefore, would strengthen tinted, bleached and waved hair by restoring hydrophobicity to the hair and decreasing the hair's porosity while bodying the hair and improving its wave properties.

Still further benefits and advantages of the present invention will become apparent to those skilled in the art from the detailed description of the invention, the examples and the claims which follow.

Disclosure of the Invention

A permanent wave neutralizer and oxidizing composition is disclosed including; (a) about 0.01 to about 10 weight percent of a water-dispersible, non-toxic polyvalent metal salt having a cation selected from the group consisting of aluminum (III), zirconium (IV), aluminum zirconium coordination complexes, and mixtures thereof; (b) about 0.5 to about 15 weight percent of an oxidizing agent; and (c) water.

Particularly preferred polyvalent metal salts are inorganic salts of a strong mineral acid. Suitable polyvalent metal salts include: aluminum chloride hexahydrate; aluminum sulfate octadecahydrate; complex basic aluminum chloride loosely associated with about 2.5 moles of water, commonly referred to as aluminum chlorohydrate; and zirconium oxychloride octahydrate. Suitable coordination complexes include aluminum chlorohydrate complexed with propylene glycol in which the water molecules normally coordinated to the aluminum chlorohydrate have been displaced by a glycol; loosely hydrated complexes of aluminum zirconium chlorohydrate, including the tri-, tetra- and pentachlorohydrates, and glycine complexes thereof; and mixtures thereof. Exemplary coordination complexes are commercially available and sold under the commercial trademark of Rehydrol II, Rezal 36G, Rezal 36GP, Rezal 67, Rezal 67P by the Reheis Chemical Company.

Suitable oxidizing agents include hydrogen peroxide, preferably present in a concentration of from about 0.5 to about 3.0 weight percent in a composition having a pH value of about 2 to about 4. Hydrogen peroxide solutions are suitably stabilized with stabilizers known in the art. The pH is adjusted with inorganic acid, preferably phosphoric acid and are prepared by methods well known in the art. The polyvalent metal salts in solution can be added to the hydrogen peroxide neutralizer immediately before use in the permanent wave procedure or preferably, the salts may be included in the stabilized hydrogen peroxide solution and stored in a single package.

Alkali-metal bromates, preferably sodium and potassium bromates, are also suitable oxidizing agents known in the art. Bromate salts are preferably present in a concentration of from about 5.0 to about 15 weight percent in a composition having a pH value of from about 4 to about 7. A person skilled in the art will appreciate that oxidizing agents useful in hair waving and hair dyeing arts, such as perborates, iodates, urea peroxide, sodium carbonate peroxide, and the like fall within the scope of this invention.

The neutralizer compositions may also include other ingredients selected from among known materials for their conventionally employed purposes. These materials can include known wetting agents, preferably of the non-ionic surface-active type, cationic surface active agents, (including cationic conditioners and cationic polymers) auxiliary conditioners, emollients, resins, fragrances, fragrance solubilizers, and the like.

In one embodiment of this invention, the neutralizer compositions are intended for use in thiol permanent waving processes that are reductivé of hair keratin disulfide bonds. The term "reductive", as used herein, also denotes the cleavage of hair disulfide bonds by sulfite salts, in the waving process sometimes referred to "sulfitolysis", since its action on the hair includes production of hair keratin thiol groups which remain in the hair during waving. The word "reductive", is meant herein to differentiate the waving procedures using thiol and sulfite salts from alkali hair treating processes that are usually carried out at high pH values, e.g., at about pH 11 to about 14. The alkali waving treatments occur primarily by the transformation of disulfide bonds in hair keratin cystine groups into lanthionine groups, while the reductive treatments are believed to occur by breaking and then reforming the hair keratin disulfide bonds. Consequently the reductive treatments require oxidation of the hair in order to rebuild the broken hair keratin disulfide bonds whereas the high alkali waving does not.

The term permanent waving as used herein includes the process of imparting a curled configuration to naturally straight hair and to the process of straightening naturally curly hair since the chemistry involved is substantially similar.

Conventional permanent waving and conventional oxidation hair tints increase the porosity of the hair and decrease the natural hydrophobicity of normal hair. The porosity of the hair can be determined by measuring its liquid retention value using well known analytical methods described below. As the porosity of the hair increases, its liquid retention value increases because the hair absorbs more water. It is known that water can swell and make the hair pliable. Thus, wet porous hair is more susceptable to stretching and breaking because of its weakened state.

Hair that has been waved and neutralized with the compositions of this invention has significantly lower liquid retention values than does hair neutralized in the usual manner. Additionally, hair neutralized with the compositions of this invention retains its natural hydrophobicity. The neutralizer compositions of this invention also restore substantial hydrophobicity to porous hair thereby strengthening it. Porous hair refers herein to hair that has been subjected to multiple tintings or to bleaches and waves (bleached/waved) with the aforementioned commercial products as described in the examples below.

The mechanism of the reaction of the polyvalent metal salts during neutralization is not clearly understood at present. It is believed the metal ion is absorbed onto and into the hair to form stable ionic bonds with the negative sites in the hair, or to form stable coordination complexes with amino nitrogen, oxygen or sulfur atoms. These metal complexes are stable to subsequent washings with conventional commercial shampoo. The metal complexes are believed to strengthen the wave configuration as well, thereby increasing the lastingness of the wave and increasing hair body. These effects are particularly beneficial to porous hair.

The stability of the metal hair complex is illustrated in the examples below using aluminum salts for convenience and are not intended to be so limited. For example, aluminum ion is not normally present in hair in detectable amounts. However, when normal hair is waved and neutralized with a composition containing 0.2 weight percent of an aluminum salt the amount of aluminum detectable in the hair after waving is from about 0.01 to about 0.03 percent. Likewise tinted hair has no detectable aluminum before waving and neutralizing with the compositions of this invention. However, after so waving the tinted hair contains from about 0.03 to about 0.11 percent aluminum. The aluminum is substantially unremoved by water rinsing and/or washings with commercial shampoo.

Hair neutralized with the compositions of this invention develops better, tighter waves that are more bodied than does hair neutralized in the conventional manner. Body is associated with greater fullness and greater volume and includes those characteristics interpreted by the consumer as springier, bouncier curls that provide a better hair style and which retain their configuration longer, hereafter referred to as set retention.

The beneficial effects previously described, especially hydrophobicity, can be achieved with a salt concentration as low as 0.01 percent. A preferred range is from about 0.05 to about 1 weight percent. Higher concentrations up to about 10 weight percent may be used but are not necessary, since the beneficial effect was not substantially augmented or enhanced beyond that achieved with lower salt concentrations.

Polyvalent metal salts of magnesium and calcium did not restore hydrophobicity to porous hair when present in a neutralizer at concentrations of from 0.2 to 20 weight percent. Similarly ineffective were monovalent ammonium and sodium salts present at concentrations of 1 weight percent as described in the examples below.

The neutralizer compositions of this invention may be in the form of a water-thin clear liquid rinse, an opaque emulsion or a cream.

This invention is further illustrated in the following examples, which are not intended to be limiting.

-10-

<u>Best Modes For Carrying Out The Invention</u>

    A.   <u>Glossary of Compounds</u>

    In the examples which follow, reference will be made to certain polyvalent and monovalent metal salts. For convenience, these salts are listed below and will be hereafter referred to by their common name or commercial trademark:

    a) Aluminum chloride is $AlCl_3 \cdot 6\,H_2O$

    b) Aluminum sulfate is $Al_2(SO_4)_3 \cdot 18H_2O$

    c) Rezal 36G is a 35% solution of a coordination complex of aluminum zirconium tetrachlorohydrate and glycine, sold under this trademark by Reheis Chemical Company.

    d) Zirconium oxychloride is $ZrOCl_2 \cdot 8\,H_2O$

    e) Chlorhydrol is a 50% solution of aluminum chlorohydrate sold under this trademark by Reheis Chemical Company.

    f) Sodium chloride is $NaCl$

    g) Ammonium sulfate is $(NH_4)_2SO_4$

    h) Magnesium sulfate is $MgSO_4 \cdot 7\,H_2O$

    i) Calcium acetate is $Ca(C_2H_3O_2)_2 \cdot H_2O$

Example 1.  Aluminum-containing Neutralizer
               Compositions

    This example illustrates hair-treating compositions containing aluminum chloride suitable for use as neutralizers in permanent wave procedures.

-11-

| | PERCENT BY WEIGHT | | | |
|---|---|---|---|---|
| Component | A | B | C | D |
| Hydrogen Peroxide (35%)[a] | 6.23 | 6.23 | 6.23 | 6.23 |
| Dicetyldimonium Chloride (75%)[b] | 1.50 | 1.50 | 1.50 | 1.50 |
| Aluminum Chloride | - | 1.00 | 0.20 | 0.20 |
| Nonoxynol-15 [c] | - | - | - | 0.50 |
| Silicone Antifoam Agent[d] | 0.01 | 0.01 | 0.01 | 0.01 |
| Water to 100g | q.s. | q.s. | q.s. | q.s. |

(a) actual hydrogen peroxide in finished formula is 2.2 weight percent.

(b) CTFA name for N-hexadecyl-N, N-dimethyl-1-hexadecanaminium chloride, such as Adogen 432CG sold under this trademark by Sherex Chemical Company, Inc., a division of Schering.

(c) CTFA name for polyoxyethylene (15) nonylphenylether, such as Igepal CO-730, sold under this trademark by GAF Corporation.

(d)CTFA name is Dimethicone (and) Silica, sold under the trademark SAG-710 by Union Carbide Corporation.

The compositions were prepared by methods well known in the art and have a pH value of about 2.5 to 3.5.

The amount of aluminum (III) in composition (B) corresponds to 0.1% and in composition (C) and (D) to 0.02%.

Compositions were prepared which were similar to compositions (A), (B) and (C), but which contained no dicetyldimonium chloride, and no silicone antifoam agent. These compositions were designated (A'), (B') and (C'), respectively.

Example 2.  Permanent Waves Neutralized With
Aluminum-containing Compositions

This example illustrates the improved wave configurations of permanent waves neutralized with the aluminum-containing composition B of Example 1 compared to its counterpart, Composition A of Example 1, containing no aluminum.

Four female volunteers having normal-type hair received permanent waves in the following half-head comparison method.  Step 1)  The hair was washed with a conventional shampoo in the usual manner.  The wet hair was wound around conventional permanent wave curlers in the usual manner.  All of the wound tresses were treated with a commercial waving lotion containing 12.8 weight/volume percent ammonium thioglycolate (calculated as thioglycolic acid), as the active reducing agent.  The waving lotion had a pH value of 7.9.  The waving lotion was maintained in contact with the hair for 20 minutes under a cap at ambient room conditions, after which time the lotion was rinsed from the wound tresses with water.  The rinsed tresses were blotted with a towel.

Step 2)  The rinsed wound tresses on one side (side A) of each of the volunteers' head was treated with neutralizer composition A; the opposite side (side B) was treated with neutralizer composition B.  After a 5-minute contact period, the wound tresses were unwound and the neutralizer was worked through the hair for about one minute.  The neutralized hair was then water-rinsed, and dried in the usual manner.

The amount of curl, and curl springiness was evaluated while the hair was wet by two experienced evaluators on the day of wave (DW) and after one shampooing one week later (1W).

-13-

Curl level is rated on a tightness scale of 1 to 10, 10 being a tight curl and 1 being no curl. Curl spring is rated on a scale of 1 to 5, 5 being the springiest when the curl is pulled straight and then released and 1 being flaccid with little or no rebound. The average results were as follows:

|  | Curl Level | | Curl Spring | |
|---|---|---|---|---|
|  | Side A | Side B | Side A | Side B |
| DW | 10.0 | 10.0 | 4.3 | 5.0 |
| 1W | 9.4 | 9.6 | 4.1 | 4.5 |

The data show that the waved side neutralized with composition B containing aluminum salt had more lasting curl spring and a longer lasting curl level than the waved side neutralized with composition A which contained no aluminum salt.

Example 3.    Permanent Waves Neutralized With
            Aluminum-Containing Composition

The procedure of Example 2 was followed in a second test using four female volunteers, except that the waving lotion used in Step 1 had a pH value of 8.2. The average results for curl level and curl spring were as follows:

|  | Curl Level | | Curl Spring | |
|---|---|---|---|---|
|  | Side A | Side B | Side A | Side B |
| DW | 10.0 | 10.0 | 4.4 | 5.0 |
| 1W | 7.8 | 8.2 | 3.3 | 3.7 |

The data again show that the waved side neutralized with composition B containing aluminum salt had better and more lasting curl spring and a longer lasting curl level than the waved side neutralized with composition A containing no aluminum.

Example 4. Permanence of Waved Curl Level and Hair
Body to Multiple "Shampoo-Set" Treatments

This example illustrates and compares the permanence of curl levels of waved hair neutralized with hair treating composition A, B, and C of Example 1 to repeated washings and settings in a "shampoo-set" relaxation procedure.

Three natural brown, virgin hair tresses (DeMeo Brothers, NY) about 6 inches in length and about 2 grams in weight were washed with a conventional shampoo, towel blotted and wound onto conventional permanent wave curlers. In a first step each wound tress was treated by saturating it with a commercial waving lotion containing 10 weight/volume percent of ammonium thioglycolate (calculated as thioglycolic acid) as the active reducing agent at a lotion pH value of 9.0. Contact was maintained with the lotion for 10 minutes under a cap at ambient room conditions after which time the lotion was rinsed from the wound tresses. Each wound tress was placed for 10 minutes under a heated salon hair dryer set at the high heat setting.

In a second step, a first wound tress was neutralized by contacting it with the hair-treating composition A of Example 1 containing no aluminum salt. Contact was maintained for 1 minute after which time the tress was unwound and the composition worked through the hair for about 5 seconds. The tress was then rinsed with water and air dried normally. A second wound tress was similarly treated, except that composition B of Example 1 was used. Likewise a third wound tress was treated, except that composition C of Example 1 was used.

In a third step, each of the above wound tresses was subjected to a "shampoo-set" relaxation procedure as follows. The wound tress was washed in the usual manner with a commercial shampoo and then wrapped around conventional "large" hair setting rollers of 1-1/8 inch ( 2.85cm) diameter. The wound tress was allowed to dry under a hair dryer.The dried tress was then unwound and combed. The "shampoo set" process was repeated two more times so that each tress received a total of 3 washings and settings. After the third treatment, the tresses were thoroughly wetted with water, combed and air dried under ambient room conditions.

Curl level permanence and hair body of each tress was subjectively evaluated by 7 experienced eval tors on a scale of 1 to 3, with 1 having the most curl or body and 3 having the least curl or body. The average results are down below:

| Neutralized With | Rank Dry Curl | Rank Hair Body |
|---|---|---|
| Composition B (1% aluminum chloride) | 1.07 | 1.57 |
| Composition C (0.2% aluminum chloride) | 1.93 | 1.43 |
| Composition A (no aluminum chloride) | 3.00 | 3.00 |

The data show that in permanent waves neutralized with a hair treating composition containing aluminum salt the waved hair had more body and longer lasting curls than did a permanent wave neutralized without aluminum salt. The data also show that curl permanence was better at 1% of aluminum chloride than at 0.2% and that bodying effects were substantially achieved with a salt concentration of 0.2%.

Example 5.    Hydrophobicity of Waved Hair
             Neutralized with Aluminum and
             Zirconium-containing Compositions

This example illustrates the ability of hair-treating neutralizer compositions containing aluminum and/or zirconium salts to restore the natural hydrophobicity of human hair normally lessened by waving treatments.

Twenty-four natural brown virgin hair tresses (DeMeo Brothers, New York) about 6-inches in length and 2 grams in weight were washed with commercial shampoo in the usual manner. The cleaned tresses were lightly blotted with a towel and each tress was wound around conventional permanent wave curlers.

One set of 16 wound tresses (tresses #1-16) was saturated with a waving lotion (F) containing 10 w/v percent thioglycolic acid as the active reducing agent at a pH value of 9.0. After a 15-minute "processing" period under a cap at ambient conditions, the tresses were rinsed with water and towel blotted. One tress (#1) was neutralized by saturating the wound tress with the hydrogen peroxide composition A' of Example 1 containing no aluminum salt. After a 5-minute "neutralizing" period, the tress was unwound and the neutralizer worked through the tress from the scalp portion to the tip portion for about 5 seconds. The neutralized tress was then rinsed with water and dried under ambient room conditions. Of the remaining 15 tresses, 13 were neutralized in the same general manner, except that the hydrogen peroxide solution above included varying amounts and types of polyvalent metal salt or monovalent alkali salt (some within the instant invention and some not), as shown below, so that tresses (#'s 2-14) individually received a different neutralizer. The remaining 2 tresses (#'s 15, 16) were neutralized in the same general manner, except that tress #15 was neutralized with an aqueous solution containing 10 weight percent sodium bromate; and tress #16 was similarly neutralized with a bromate solution including 1 weight percent aluminum chloride added immediately prior to use.

In a second experiment, a set of 4 wound tresses (#'s 17-20) were treated with waving lotion (F) following the above procedure, except that the "processing" period was shortened to 5 minutes. One tress (#17) was neutralized with composition A' of Example 1 following the above procedure. The remaining tresses (#'s 18, 19, 20) were similarly neutralized with the above peroxide solution, except that the peroxide included the varying amounts aluminum chloride shown below so that tresses #'s 18, 19, 20 individually received a different neutralizer.

In a third experiment, a set of 2 wound tresses (#21, 22) was waved with a waving lotion (H) containing glycerylthioglycolate (12 w/v percent as active thioglycolic acid) as the active reducing agent at a pH value of 6.9. The product directions were followed and the tresses were treated with the waving lotion for 20 minutes under a heated salon hair dryer using the high setting. Tress #21 was neutralized as above with the peroxide solution A' of Example 1 and tress #22 was neutralized with peroxide solution B' of Example 1 including 1 weight percent aluminum salt.

-18-

In a fourth experiment, a set of 2 wound tresses (#'s 23, 24) were waved with a commercial waving lotion (J) containing 9 weight percent ammonium bisulfite as the active reducing agent at a pH value of 6.9. The product directions were followed and the tresses were treated with the waving lotion for 20 minutes under a heated salon hair dryer. Tress #23 was neutralized with peroxide solution A' of Example 1 and tress #24 was neutralized with aqueous peroxide solution B' of Example 1 as above.

The hydrophobicity of each of the above tresses was evaluated using the following float/sink test. The results are in Table 1.

Float/Sink Test

Dry hair fibers were cut to about 1 cm in length and weighed to about 0.1 gram. The hair fibers were spread out uniformly on a piece of glazed paper within a circle of about 1.5 inch (3.81 cm.) in diameter. The fiber mass was placed gently on the surface of water (about 90 ml of water in a 100 ml beaker) and the paper carefully removed leaving the fiber mass floating on the surface. The length of time the fiber mass remained floating before sinking into the water was recorded.

## TABLE I

Hydrophobicity of Waved Hair Neutralized with and without Metal Salt

| Tress | Waving Lotion Ammonium Thioglycolate pH 9.0 (Lotion F) | (Weight Percent) Metal Salt in Neutralizer Hydrogen Peroxide (2.2%) with | | Hydrophobic Floating Time |
|---|---|---|---|---|
| 1 | 15 min. process | None (Comp. A', Ex. 1) | | 5 seconds |
| 2 | 15 min. process | Aluminum Chloride | (0.2%) | 24 hours+ |
| 3 | 15 min. process | " " | (0.5%) | 24 hours+ |
| 4 | 15 min. process | " " | ( 1% ) | 24 hours+ |
| 5 | 15 min. process | " " | ( 5% ) | 1 hour |
| 6 | 15 min. process | " " | (10% ) | 1 hour |
| 7 | 15 min. process | Aluminum Sulfate | ( 1% ) | 24 hours+ |
| 8 | 15 min. process | Rezal 36G | ( 1% ) | 24 hours+ |
| 9 | 15 min. process | Zirconium Oxychloride | ( 1% ) | 24 hours+ |
| 10 | 15 min. process | Chlorhydrol | ( 2% ) | 24 hours+ |
| 11 | 15 min. process | Sodium Chloride | ( 1% ) | 2 seconds |
| 12 | 15 min. process | Ammonium Sulfate | ( 1% ) | 1 second |
| 13 | 15 min. process | Magnesium Sulfate | ( 1% ) | 3 seconds |
| 14 | 15 min. process | Calcium Acetate | ( 1% ) | 3 seconds |
| | Ammonium Thioglycolate pH 9.0 (Lotion F) | Sodium Bromate (10%) with | | |
| 15 | 15 min. process | None | | 2 seconds |
| 16 | 15 min. process | Aluminum Chloride | ( 1% ) | 24 hours |

(Weight Percent)

| Tress | Waving Lotion | Metal Salt in Neutralizer | Hydrophobic Floating Time |
|---|---|---|---|
| | Ammonium Thioglycolate pH 9.0 (Lotion F) | Hydrogen Peroxide (2.2%) with | |
| 17 | 5 min. process | None (Comp. A', Ex. 1) | 15 seconds |
| 18 | 5 min. process | Aluminum Chloride (0.05%) | 24 hours |
| 19 | 5 min. process | Aluminum Chloride (0.1% ) | 24 hours |
| 20 | 5 min. process | Aluminum Chloride (0.2% ) | 24 hours |
| 21 | Glyceryl Thioglycolate pH 6.9 (Lotion H) 20-min. dryer process | None (Comp. A', Ex. 1) | 5 seconds |
| 22 | " " | Aluminum Chloride ( 1% ) | 24 hours |
| 23 | Ammonium Bisulfite pH 6.9 (Lotion J) 20-min dryer process | None (Comp. A', Ex. 1) | 1 minute |
| 24 | " " | Aluminum Chloride ( 1% ) | 24 hours |
| | For Comparison: | | |
| 25 | Virgin Hair Control, not waved. | -- | 24 hours |

-21-

The data show that normal virgin hair is naturally hydrophobic (Tress 25) and that it loses this characteristic when it is waved with conventional products and neutralized with conventional hair-treating solutions (Tresses 1, 15, 17, 21, 23) containing no aluminum or zirconium salt. Neutralizer solutions containing aluminum and zirconium metal salts substantially restored the hydrophobic character to the waved hair (Tresses 2-10, 16, 18-20, 22, 24) whereas polyvalent magnesium and calcium salts and the monovalent sodium and ammonium salts did not (Tresses 11-14).

It is to be noted that hydrophobicity was substantially the same for tresses neutralized with compositions containing 5 and 10% aluminum salt and that preferred results were effectively achieved at aluminum salt concentrations below about 5%. The mechanism of this effect is not fully understood. However, it is believed that normal hair, following a single permanent wave as practiced herein, is substantially strengthened at salt concentrations below 5%, more preferably from about 0.05 to about 1%.

Example 6. Hydrophobicity of Tinted and Waved Hair Neutralized With Aluminum-containing Compositions

This example illustrates the ability of a hair-treating neutralizer composition containing aluminum salt to restore hydrophobicity to tinted hair when it is subsequently waved and neutralized.

Tinted hair was prepared by dyeing 8 naturally dark brown tresses of normal human hair (DeMeo Brothers, New York) about 6-inches in length and 2 grams in weight. The tresses were dyed for 25 minutes using a commercial oxidative-type hair dye of a light hair shade following the product directions. The dyed tresses were washed with a commercial shampoo in the usual manner at the end of the coloring procedure. The tresses were redyed as above two more times for a total of 3 dye treatments. The resulting hair color was light brown.

Seven of the tinted hair tresses (#1-7) were again washed with shampoo as above, towel blotted and then each wound around conventional permanent wave curlers. In a first step each of the wound tresses was saturated with a commercial waving lotion containing 10 w/v percent ammonium thioglycolate (as thioglycolic acid) as the active reducing agent at a pH value of 8.2. After a 10-minute "processing" period, each tress was rinsed with water and towel blotted. In a second step, one of the waved tresses (#1) was neutralized by saturating the wound tress with the aqueous peroxide solution A' of Example 1. After a 5-minute "neutralizing" period, the tress was unwound and the neutralizer rubbed through the tress from the scalp to tip portion. The neutralized tress was then rinsed with water and dried under ambient room conditions. Each of the remaining tresses was similarly neutralized in the same general manner, except that the hydrogen peroxide solution included varying amounts of polyvalent metal salts as shown below so tresses (#2-7) individually received a different neutralizer.

The hydrophobicity of each tinted/waved tress was evaluated following the Float/Sink Test of Example 5. The results are shown below:

| Tress | (Weight Percent) Metal Salt in Neutralizer | | Hydrophobic Floating Time |
|---|---|---|---|
| 1 | None (Comp. A', Exam. 1) | | 1 second |
| 2 | Aluminum Chloride | (0.2%) | 1 hour+ |
| 3 | Aluminum Sulfate | (0.2%) | 1 hour+ |
| 4 | Magnesium Sulfate | (0.2%) | 1 second |
| 5 | Calcium Acetate | (0.2%) | 1 second |
| 6 | Magnesium Sulfate | (20% ) | 1 second |
| 7 | Calcium Acetate | (20% ) | 1 second |
| | For Comparison | | |
| 8 | Tinted Hair Control Not Waved | | 1 second |

The results show that tinted hair, by itself, was not hydrophobic (tress #8) and that a conventional wave treatment did not restore any hydrophobicity to the tinted hair (tress #1). However, when the tinted hair was waved and then neutralized with a composition containing 0.2 weight percent aluminum salt, substantial hydrophobicity was restored (tress #'s 2, 3), although not to the level of virgin (untinted, unwaved) hair (Tress #25 of Example 5). Neutralizer compositions containing magnesium or calcium as the polyvalent metal cation, restored no hydrophobicity to tinted hair at salt concentrations of 0.2 weight percent or even when the salt content was increased 100 fold (tress #'s 4, 5, 6, and 7).

Example 7. Porosity of Tinted and Waved Hair Neutralized with Aluminum-containing Compositions

This example illustrates the improvement in the porosity of tinted and waved hair (tinted/waved) that is neutralized with a hair-treating composition containing aluminum chloride relative to that of similarly tinted and waved hair neutralized without aluminum salt.

Three tinted hair tresses were prepared following the procedure of Example 6. The three tinted tresses were waved using the waving lotion of Example 6 and the waving and neutralization procedure of Example 4, except that tress #1 was neutralized with the aqueous peroxide solution B of Example 1, tress #2 was neutralized with composition C of Example 1 and tress #3 was neutralized with composition A of Example 1, and the total neutralizing period was 1 minute before unwinding the tresses.

The porosity of the tinted/waved hair was measured relative to its water absorption using a method for determining liquid retention values similar to that described by Wolfram and Underwood in Textile Research Journal 36(11), pp. 947-953 (1966) incorporated herein by reference. Under this method, tinted hair which has not been waved has liquid retention value of about 40%. The data were as follows:

| Tress | Neutralized with | % Liquid Retained (Average of 4 determinations ) |
|-------|------------------|--------------------------------------------------|
| 1 | Composition B (1% aluminum chloride) of Example 1 | 45.27 |
| 2 | Composition C (0.2% aluminum chloride) of Example 1 | 47.99 |
| 3 | Composition A (no aluminum chloride) of Example 1 | 52.33 |

A statistical analyses of the results using the well known Student's t test showed that hair neutralized with either composition B or C of Example 1 had significantly lower liquid retention values that the hair neutralized with composition A of Example 1. Thus the aluminum salt strengthened the tinted/waved hair by leaving the hair in a less porous state than did a conventional treatment without aluminum salt.

Example 8.    Deposition of Aluminum Inside the
             Hair During a Waving Process

This example illustrates the deposition of aluminum inside the hair during a waving process when an aluminum salt-containing neutralizer was used. It also illustrates the substantivity of the deposited aluminum in hair toward subsequent shampoo.

In one experiment a set of 5 tinted tresses were prepared following the method of Example 6. The tinted tresses were waved with the waving lotion and procedure of the first step of Example 6. In a second step, one tress (A) was neutralized following the 5-minute procedure of Example 6 except that composition  D  of Example 1 was used. The treated tress was analyzed for percent aluminum content by an atomic absorption method.

A second tress (B) was neutralized as above and then washed with a commercial shampoo in the usual manner and then analyzed as above. A third tress (C) was treated in the same manner as tress (A), except that the treatment was repeated twice so that the hair was waved a total of 3 times. A fourth tress (D) was treated in the same manner as tress (B) except that the treatment was repeated twice for a total of 3 wavings and 3 washings.

0134452

-26-

In a second experiment, the above procedure was followed except that natural brown normal hair (DeMeo Brothers, New York) and composition C of Example 1 was used and the lotion had a pH of 9.0. The results of the aluminum analysis for both experiments is shown below compared to untreated control hair (E).

| | | % Aluminum in Hair | |
|---|---|---|---|
| | | Virgin | Tinted |
| A) | 1 X Waved | 0.02 | 0.05 |
| B) | 1 X Waved & Shampooed | 0.01 | 0.03 |
| C) | 3 X Waved | 0.03 | 0.11 |
| D) | 3 X Waved & Shampooed | 0.02 | 0.10 |
| E) | Unwaved control | Not detectable | Not detectable |

The data show that unwaved normal and tinted hair has no detectable aluminum. Aluminum deposited in the hair during the neutralization step of the waving process of this invention. The data further show that the deposited aluminum was substantive to the hair, since it was not readily removed by washing with shampoo. Multiple wavings increased the amount of aluminum in the hair, especially in tinted hair.

The present invention has been described with respect to preferred embodiments. It will be clear to those skilled in the art that modifications and/or variations of the disclosed compositions and methods can be made without departing from the scope of the invention set forth herein. The invention is defined by the claims that follow.

WHAT IS CLAIMED IS:

1. A permanent wave neutralizer and oxidizing composition including:

(a) about 0.01 to about 10 weight percent of a water-dispersible, non-toxic polyvalent metal salt of a strong mineral acid having a cation selected from the group consisting of aluminum (III), zirconium (IV), aluminum zirconium coordination complexes and mixtures thereof;

(b) about 0.5 to about 15 weight percent of an oxidizing agent;

(c) water;

wherein the composition has a pH value of about 2 to about 7.

2. The composition of claim 1 wherein the metal salt is an aluminum (III) salt selected from the group consisting of aluminum chloride, aluminum sulfate and mixtures thereof.

3. The composition of claim 1 wherein the metal salt is aluminum chlorohydrate.

4. The composition of claim 1 wherein the metal salt is zirconium (IV) oxychloride.

5. The composition of claim 1 wherein the metal salt is a loosely hydrated coordination complex of aluminum zirconium chlorohydrate selected from the group consisting of tri-, tetra- and pentachlorohydrates and glycine complexes thereof.

6. The composition of claim 1 wherein the metal salt is a propylene glycol coordinate complex of aluminum chlorohydrate.

7. A permanent wave neutralizer and oxidizing composition including

(a) about 0.05 to about 1 weight percent of a water-dispersible non-toxic polyvalent metal salt of a strong mineral acid having a cation selected from the group consisting of aluminum (III), zirconium (IV), aluminum zirconium coordination complexes and mixtures thereof;

(b) about 0.5 to about 15 weight percent of an oxidizing agent; and

(c) water;

wherein the composition has a pH value of about 2 to about 7.

8. The composition of claim 7 wherein the oxidizing agent is hydrogen peroxide present at a concentration of about 0.5 to 3.0 weight percent in a composition having a pH value of about 2 to about 4.

9. The composition of claim 7 wherein the oxidizing agent is sodium bromate present at a concentration of about 5 to about 15 weight percent in a composition having a pH value of about 4 to about 7.

10. A method of treating hair for purposes of permanently waving said hair comprising the steps of contacting the hair with an effective amount of hair keratin disulfide-bond breaking agent selected from the group consisting of thiol and sulfite reducing agents; maintaining said contact for a period of time sufficient to form partially reduced hair keratin; removing the keratin reducing agent from the hair; contacting the partially reduced hair with the neutralizer composition of claim 1; maintaining said contact for a period of time sufficient to rebuild the broken keratin disulfide bonds; and removing the neutralizer from the hair.

11. A method of treating hair for purposes of permanently waving said hair comprising the steps of contacting the hair with an effective amount of hair keratin disulfide-bond breaking agent selected from the group consisting of thiol and sulfite reducing agents; maintaining said contact for a period of time sufficient to form partially reduced hair keratin; removing the keratin reducing agent from the hair; contacting the partially reduced hair with the neutralizer composition of claim 7; maintaining said contact for a period of time sufficient to rebuild the broken keratin disulfide bonds; and removing the neutralizer from the hair.